# FASCICULE DE BREVET EUROPEEN

(11) **EP 3 727 592 B1**
(45) Date de publication et mention de la délivrance du brevet: **01.02.2023**
(21) Numéro de dépôt: 18842544.1
(22) Date de dépôt: 20.12.2018
(51) Int. Cl.: A61Q 1/02, A61Q 1/04, A61Q 1/06, A61K 8/92, A61K 8/02, A61K 8/31, A61K 8/89

(54) **COMPOSITIONS COSMÉTIQUES COMPRENANT DES SPHEROIDES ANHYDRES DISPERSÉS DANS UNE PHASE SILICONÉE**
KOSMETISCHE ZUSAMMENSETZUNGEN MIT IN EINER SILIKONPHASE DISPERGIERTEN WASSERFREIEN SPHÄROIDEN
COSMETIC COMPOSITIONS COMPRISING ANHYDROUS SPHEROIDS DISPERSED IN A SILICONE PHASE

(30) Priorité: 22.12.2017 FR 1763086
(43) Date de publication de la demande: 28.10.2020
(73) Titulaire: LVMH Recherche, 45800 Saint Jean de Braye (FR)
(72) Inventeur: DE LA POTERIE, Valérie, 45740 LAILLY EN VAL (FR); MANIGUET, Sabrina, 45470 TRAINOU (FR); MASANELLI, Armelle, 45510 TIGY (FR)
(74) Mandataire: Cabinet Beau de Loménie
(86) Numéro de dépôt international: PCT/FR2018/053462
(87) Numéro de publication internationale: WO 2019/122754

(56) Documents cités:
- EP-A1- 0 452 202
- EP-A1- 0 665 008
- EP-A2- 0 462 709
- FR-A1- 2 649 608
- FR-A1- 2 779 962

## Description

L'invention porte sur une composition cosmétique comprenant des sphéroïdes anhydres en dispersion dans une phase continue non miscible comprenant au moins une huile de silicone. Plus particulièrement, cette composition cosmétique peut être un rouge à lèvre ou un fond de teint.

### ETAT DE L'ART ANTÉRIEUR ET BUT DE L'INVENTION

L'industrie cosmétique est depuis toujours à la recherche de compositions produisant des effets surprenants. Il peut s'agir de compositions ayant un aspect visuel original, par exemple en dispersant des particules solides de différentes couleurs dans une phase continue transparente. Il peut également s'agir de nouvelles compositions combinant des performances difficilement associables par des techniques classiques, telles que fraîcheur et brillance, fraîcheur et tenue, brillance et tenue.

Toutefois, l'obtention de compositions stables par association de phases non miscibles n'est pas chose aisée, l'incompatibilité entre les phases posant des problèmes de stabilité.

Dans le cas d'émulsions combinant deux phases liquides, l'homme du métier va chercher à éviter que les globules de la phase dispersée fusionnent, et ainsi conduisent à une séparation des phases, par exemple en utilisant des agents tensioactifs pour stabiliser les gouttelettes.

Dans le cas de dispersions de particules solides visibles dans une phase continue liquide ou gélifiée, les particules solides sont souvent soit dispersées, soit en suspension dans la phase continue. Il est particulièrement important d'éviter une diffusion progressive de la phase continue dans les particules solides ou, à l'inverse, une diffusion du matériau constituant les particules solides dans la phase continue. Ceci est particulièrement problématique lorsque la phase continue est translucide ou transparente, car toute diffusion de matière dans la phase continue est alors immédiatement visible.

Un premier problème à la base de la présente invention consiste ainsi à obtenir une composition anhydre stable sous la forme de sphéroïdes solides dispersés dans une phase continue, dans laquelle les sphéroïdes sont suffisamment durs à température ambiante pour conserver leur forme et leur intégrité, ne se déforment pas, et ne relarguent pas de matière dans ladite phase continue non miscible avec lesdits sphéroïdes.

Un deuxième problème à la base de l'invention est d'obtenir une composition biphasique dans laquelle les sphéroïdes présentent une structure suffisamment souple pour être facilement écrasés et appliqués sur la peau par un léger mouvement de cisaillement ou d'écrasement, par exemple à l'aide d'un doigt. Les sphéroïdes peuvent ainsi être facilement mélangés avec la phase continue pour ne former qu'une seule phase au moment de l'application, par exemple sur la peau ou les lèvres, et ainsi combiner les propriétés des deux phases.

Des compositions comprenant des sphéroïdes dans une phase continue ont déjà été décrites dans l'art antérieur.

FR 2 649 608 décrit par exemple une composition comprenant une phase continue sous forme de gel aqueux contenant, en suspension dans ladite phase continue, des sphéroïdes lipidiques ayant un diamètre moyen compris entre 50 et 10000 µm et ayant un point de fusion inférieur à 50°C. Les sphéroïdes de bas point de fusion présentent une dureté insuffisante qui affecte à la fois leur forme (sphéroïdes polydisperses et non homogènes) et la stabilité de la composition finale.

Les Inventeurs ont observé que des compositions cosmétiques anhydres comprenant des sphéroïdes constitués d'une matrice lipidique présentant un point de fusion supérieur à 50°C, dispersés dans une phase continue comprenant au moins une huile de silicone, permettent de résoudre de façon tout à fait surprenante les problèmes exposés ci-dessus.

De façon inattendue, ils ont aussi mis en évidence que les sphéroïdes de haut point de fusion présentent un bon étalement. Ainsi, les compositions anhydres de l'invention s'appliquent très facilement et produisent une texture surprenante au moment de l'application sur la peau, les sphéroïdes et la phase continue non miscible dans laquelle ils sont dispersés se mélangeant *in situ* pour accentuer une performance sensorielle et/ou colonelle, comme par exemple une propriété de brillance, de tenue, de sensorialité ou de confort à la surface de la peau. On peut observer la transformation des compositions selon l'invention de gel en crème et/ou la transformation d'un mélange de sphéroïdes de différentes couleurs avec la phase continue en une seule teinte uniforme, tout en produisant un effet sensoriel surprenant du fait du contact simultané de ces deux phases non miscibles avec la peau.

A ce jour, il n'a jamais été proposé de compositions anhydres dont les phases non miscibles permettent de résoudre les problèmes mentionnés ci-dessus.

### DESCRIPTION DE L'INVENTION

Selon un premier aspect, la présente invention a pour objet une composition cosmétique anhydre comprenant des sphéroïdes anhydres ayant un diamètre de 0,1 à 10 mm et présentant un point de fusion supérieur à 50°C, lesdits sphéroïdes étant constitués d'une matrice lipidique cosmétiquement acceptable comprenant :
(a) au moins une huile hydrocarbonée, et éventuellement un agent gélifiant de ladite huile hydrocarbonée, et/ou
(b) au moins une cire, lesdits sphéroïdes étant en dispersion dans une phase continue non miscible comprenant au moins une huile de silicone.

Au sens de l'invention, on entend par « matrice lipidique » une composition homogène (c'est-à-dire ne comprenant pas une couche d'enrobage autour d'un noyau) à base de substances grasses, c'est-à-dire comprenant (a) au moins une huile hydrocarbonée, et/ou (b) au moins une cire.

Au sens de l'invention, l'expression « non miscible » signifie que les sphéroïdes de l'invention ne se diffusent pas, ne se délitent pas et ne gonflent pas, dans la phase dans laquelle ils sont mis en dispersion.

Au sens de l'invention, le terme « sphéroïde » vise un petit solide de forme essentiellement sphérique, solide à température ambiante, ayant la même composition dans tout le sphéroïde. On entend par température ambiante, une température comprise entre 15 et 27°C, et plus généralement entre 20 et 25°C. Le diamètre des sphéroïdes peut varier de 0,1 à 10 mm, de préférence de 0,3 à 8 mm, plus préférentiellement de 0,5 à 5 mm, et encore plus préférentiellement de 1 à 3 mm, ce diamètre étant un diamètre moyen mesuré sur dix mesures par des méthodes classiques, par exemple à l'aide d'une loupe binoculaire ou d'un tamis. Ces sphéroïdes ont de préférence un aspect régulier, une surface lisse et un volume uniforme.

Avantageusement, les sphéroïdes de l'invention sont exempts de tout enrobage externe visant à isoler la matrice lipidique du milieu externe (*i.e.* la phase continue), ayant une composition différente de celle de la matrice lipidique.

Le terme « anhydre » signifie, dans le cadre de l'invention, que la teneur en eau de l'objet considéré est de préférence inférieure à 1% en poids, et encore plus préférentiellement inférieure 0,5% en poids, dudit objet.

Les sphéroïdes de l'invention sont constitués d'une matrice lipidique dont la texture, à température ambiante, est suffisamment souple et déformable pour pouvoir être appliquée facilement sur la peau via un faible cisaillement, par exemple avec les doigts, et ainsi produire un effet de soin ou de maquillage sur la peau.

Les Inventeurs ont observé que la nature des constituants entrant dans la composition des sphéroïdes de l'invention peut avoir une influence non seulement sur la facilité d'application de ces sphéroïdes, mais également sur leurs propriétés finales.

Ainsi, selon un mode de réalisation avantageux, l'huile hydrocarbonée (a) est choisie parmi les huiles hydrocarbonées volatiles et/ou non volatiles. De préférence, il s'agit d'un mélange d'huiles hydrocarbonées.

La ou les huiles hydrocarbonées (a), et éventuellement l'agent gélifiant de ladite huile hydrocarbonée, peuvent être présentes à une teneur allant de 5 à 99,5% en poids, de préférence de 10 à 95% en poids, plus préférentiellement de 20 à 90%, encore plus préférentiellement de 30 à 85% en poids, et encore plus préférentiellement de 40 à 80% en poids, par rapport au poids total de la matrice lipidique.

Au sens de l'invention, on entend par « huile hydrocarbonée » une huile contenant principalement des atomes de carbone et d'hydrogène, et éventuellement des atomes d'oxygène, d'azote, de soufre, de phosphore.

Une huile hydrocarbonée volatile au sens de l'invention est une huile susceptible de s'évaporer au contact de la peau en moins d'une heure, à température ambiante et pression atmosphérique. La ou les huiles volatiles de l'invention sont des huiles liquides à température ambiante, ayant une pression de vapeur non nulle, à température ambiante et pression atmosphérique, allant en particulier de 0,13 Pa à 40 000 Pa (10⁻³ à 300 mm de Hg), en particulier allant de 1,3 Pa à 13 000 Pa (0,01 à 100 mm de Hg), et plus particulièrement allant de 1,3 Pa à 1300 Pa (0,01 à 10 mm de Hg). Par huile hydrocarbonée non volatile, on entend une huile restant sur la peau à température ambiante et pression atmosphérique au moins plusieurs heures et ayant notamment une pression de vapeur inférieure à 0,13 Pa (10⁻³ mm de Hg).

Les huiles hydrocarbonées volatiles de l'invention sont avantageusement choisies parmi les huiles hydrocarbonées comprenant de 8 à 16 atomes de carbone, et notamment les alcanes ramifiés comprenant de 8 à 16 atomes de carbone comme les isoalcanes comprenant de 8 à 16 atomes de carbone (appelées aussi isoparaffines) d'origine pétrolière comme l'isododécane (encore appelé 2,2,4,4,6-pentaméthylheptane), l'isodécane, l'isohexadécane, et par exemple les huiles vendues sous les noms commerciaux d'Isopar^{™} ou de Permetyls (ExxonMobil Chemical), les esters ramifiés comprenant de 8 à 16 atomes de carbone comme le néopentanoate d'iso-hexyle, et leurs mélanges. D'autres huiles hydrocarbonées volatiles comme les distillats de pétrole, notamment ceux vendus sous la dénomination Shell Soit par la société SHELL, peuvent aussi être utilisées. Les huiles hydrocarbonées volatiles peuvent également être choisies parmi les alcanes linéaires comprenant de 8 à 16 atomes de carbone. A titre d'exemple d'alcane linéaire comprenant de 8 à 16 atomes de carbone, on peut citer le n-nonadécane (C₉), le n-décane (C₁₀), le n-undécane (C₁₁), le n-dodécane (C₁₂), le n-tridécane (C₁₃), le n-tetradécane (C₁₄), le n-pentadécane (C₁₅), le n-hexadécane (C₁₆), et leurs mélanges, et en particulier le mélange de n-undécane (C₁₁) et de n-tridécane (C₁₃) commercialisé sous la référence de CETIOL UT par la Société Cognis.

Selon un mode de réalisation, un alcane linéaire volatil convenant à l'invention peut être choisi parmi le n-nonadécane, le n-undécane, le n-dodécane, le n-tridécane, et leurs mélanges.

Les huiles hydrocarbonées volatiles de l'invention sont avantageusement choisies parmi les huiles volatiles hydrocarbonées comprenant de 8 à 16 atomes de carbone, et leurs mélanges.

Comme huiles hydrocarbonées non volatiles, on peut notamment citer : les huiles hydrocarbonées d'origine végétale telles que les triesters d'acides gras et de glycérol dont les acides gras comprenant de 4 à 24 atomes de carbone, ces huiles pouvant être linéaires ou ramifiées, saturées ou insaturées. Ces huiles sont avantageusement des huiles de germe de blé, de tournesol, de pépins de raisin, de sésame, de maïs, d'abricot, de ricin, de karité, d'avocat, d'olive, de soja, d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat ; ou encore des triglycérides d'acides caprylique/caprique comme ceux vendus par la société Stéarineries Dubois ou ceux vendus sous les dénominations Miglyol^{®} 810, 812, 818 et 829 par la société Dynamit Nobel ; ou encore des hydrocarbures linéaires ou ramifiés, comprenant de 4 à 24 atomes de carbone, d'origine minérale ou synthétique tels que la vaseline, les polydécènes, le polyisobutène hydrogéné tel que le Parleam^{®}, le squalane, et leurs mélanges ; les esters de synthèse comme par exemple l'huile de Purcellin^{™} (octanoate de cétostéaryle), le myristate d'isopropyle, le palmitate d'isopropyle, le benzoate d'alcool comprenant de 12 à 15 atomes de carbone, le laurate d'hexyle, l'adipate de diisopropyle, l'isononanoate d'isononyle, le palmitate de 2-éthyl-hexyle, l'isostéarate d'isostéarate, des octanoates, décanoates ou ricinoléates d'alcools ou de polyalcools comme le dioctanoate de propylène glycol ; les esters hydroxylés comme le lactate d'isostéaryle, le malate de di-isostéaryle ; et les esters du pentaérythritol ; les alcools gras liquides à température ambiante, à chaîne carbonée ramifiée et/ou insaturée comprenant de 12 à 26 atomes de carbone comme l'octyl dodécanol, l'alcool isostéarylique, l'alcool oléique, le 2-hexyldécanol, le 2-butyloctanol, le 2-undécylpentadécanol ; les acides gras supérieurs tels que l'acide oléique, l'acide linoléique, l'acide linolénique ; les carbonates, les acétales, les citrates, et leurs mélanges.

Lorsque la matrice lipidique de l'invention ne comprend pas de cire (b), la ou les huiles hydrocarbonées (a) sont combinées à au moins un agent gélifiant desdites huiles hydrocarbonées.

L'agent gélifiant de l'huile hydrocarbonée est de préférence choisi parmi les silices, les argiles, les hectorites éventuellement modifiées, les esters de dextrine, les polyamides ou les polyamides siliconés, les amides de l'acide L-glutamique ou de l'acide aspartique, les copolymères blocs hydrocarbonés comprenant au moins une unité styrène.

Au sens de l'invention, les hectorites peuvent être des hectorites modifiées par un chlorure d'alkylammonium quaternaire, de préférence un ammonium substitué par au moins un, de préférence au moins deux groupements alkyles comprenant de 14 à 20 atomes de carbone. L'alkyle peut avantageusement être le stéaryle. On citera le composé portant le nom INCI distéardimonium hectorite dans lequel l'ammonium comprend deux groupes méthyles et deux groupes stéaryles.

Au sens de l'invention, les esters de dextrine sont des esters de dextrine et d'acide gras comprenant de 12 à 24 atomes de carbone, de préférence comprenant de 14 à 22 atomes de carbone, et encore plus préférentiellement comprenant de 14 à 18 atomes de carbone. De préférence, les esters de dextrine sont choisis parmi le myristate de dextrine, le palmitate de dextrine, et leurs mélanges.

Au sens de l'invention, les amides de l'acide L-glutamique (glutamides) ou de l'acide aspartique comprennent de préférence au moins un groupement alkoyle comprenant de 6 à 14 atomes de carbone, par exemple 8 ou 12 atomes de carbone. Un tel amide de l'acide glutamique est par exemple décrit dans le brevet FR 2 820 739. Les glutamides sont de préférence choisis parmi le dibutyl lauroyl glutamide, le dibutyl éthylhexanoyl glutamide, et leurs mélanges, et peut être par exemple un des produits de marques EB-21, GP-1, AJK-OD2046, AJK-BG2055 et AJK-CE2046 fabriqués par la société Ajinomoto. Le glutamide est par exemple choisi parmi le dibutyl lauroyl glutamide et le dibutyl éthylhexanoyl glutamide, ou leurs mélanges.

L'amide ou le mélange d'amides, notamment de glutamide(s), représente par exemple entre 0,1% et 15,0% en poids, entre 1,0% et 15,0 % en poids, entre 0,5% et 8,0% en poids, entre 1,0% et 5,0% en poids, entre 0,8% et 5% en poids, voir entre 2,0 et 3,0% en poids par rapport au poids total de la matrice lipidique.

Au sens de l'invention, les copolymères blocs hydrocarbonés comprenant au moins une unité styrène, sont de préférence des copolymères blocs de styrène et d'oléfine, comme par exemple les copolymères comprenant au moins une unité styrène et un motif choisi parmi le butadiène, l'éthylène, le propylène, le butylène, l'isoprène, et leurs mélanges. Les copolymères blocs hydrocarbonés de l'invention sont avantageusement choisis parmi les copolymères de styrène-éthylène/propylène, les copolymères de styrène-éthylène/propylène-styrène, les copolymères de styrène-éthylène/butadiène-styrène, les copolymères de styrène-isoprène-styrène, les copolymères styrène-butadiène-styrène, les copolymères styrène-éthylène/butylène-styrène, les copolymères styrène/méthylstyrène/indène, et leurs mélanges.

La ou les cires (b) utilisées dans le cadre de l'invention peuvent être un composé lipophile, solide à température ambiante, à changement d'état solide/liquide réversible, ayant une température de fusion supérieure à 30°C, de préférence de 50 à 120°C, plus préférentiellement de 60 à 110°C, et encore plus préférentiellement de 70 à 100°C. Au sens de l'invention, la température de fusion correspond à la température du pic le plus endothermique observé en analyse thermique (DSC) telle que décrite dans la norme NF EN ISO 11357-3. La température de fusion de la cire peut être mesurée à l'aide d'un calorimètre à balayage différentiel (DSC), par exemple le calorimètre vendu sous la dénomination DSC Mettler Toledo.

On peut avantageusement former des mélanges de cires en combinant des cires à haute température de fusion, c'est-à-dire dont la température de fusion est supérieure à 50°C, de préférence supérieure à 70°C, avec des cires ayant des températures de fusion plus basses, c'est-à-dire dont la température de fusion est inférieure à 50°C, de préférence inférieure ou égale à 40°C. Le mélange de cires ayant une haute température de fusion avec des cires ayant une basse température de fusion doit permettre l'obtention de sphéroïdes présentant un point de fusion supérieur à 50°C.

Les sphéroïdes de l'invention peuvent quant à eux avantageusement être caractérisés par un point de fusion (ou point de goutte) allant de 50 à 120°C, plus préférentiellement de 60 à 110°C ; et encore plus préférentiellement allant de 70 à 100°C. Le point de fusion de la matrice lipidique correspond à la température à laquelle apparaît la première goutte de matrice lipidique lorsque celle-ci est chauffée.

La ou les cires (b) utilisées dans le cadre de l'invention peuvent être choisies parmi les cires solides à température ambiante d'origine animale, végétale, minérale ou de synthèse, et leurs mélanges.

Avantageusement, la ou les cires (b) de l'invention peuvent être choisies parmi :
- les cires hydrocarbonées comme la cire d'abeille, la cire de lanoline, les cires d'insectes de Chine, la cire de son de riz, la cire de Carnauba, la cire de Candellila, la cire d'Ouricury, la cire d'Alfa, la cire de fibres de liège, la cire de canne à sucre, la cire de berry, la cire de shellac, la cire du Japon, la cire de sumac, la cire de montan, les cires d'orange et de citron, les paraffines et l'ozokérite, les cires de polyméthylène, de polyéthylène, de polypropylène et leurs copolymères éthylène/propylène, les cires obtenues par hydrogénation catalytique d'huiles animales ou végétales ayant des chaînes grasses, linéaires ou ramifiées, comprenant de 8 à 22 atomes de carbone, comme l'huile de jojoba isomérisée, l'huile de tournesol hydrogénée, l'huile de ricin hydrogénée, l'huile de coprah hydrogénée, l'huile de lanoline hydrogénée, le tétrastéarate de di-(triméthylol-1,1,1-propane) et le tétrabéhénate de di-(triméthylol-1,1,1-propane) ;
- les cires d'alcools gras choisies parmi les alcools gras saturés ou insaturés, linéaires ou ramifiés, comprenant de 20 à 60 atomes de carbone,
- les cires de silicone, comme les alkyl- ou alkoxy-diméthicones comprenant de 16 à 45 atomes de carbone, ou les cires fluorées, et
- leurs mélanges.

Selon un mode de réalisation particulièrement avantageux, la ou les cires (b) sont des cires apolaires, de préférence choisies parmi les cires d'hydrocarbures telles que la Microcrystalline Wax SP-88 et la Microcrystalline Wax SP-16 W (Strahl and Pitsch Inc.), et/ou les cires de polyéthylène telles que les cires Jeenate^{®} (Jeen International Corporation) et Performalene^{®} (Baker Hughes), les cires d'hydrocarbures étant les plus préférées. Les cires d'hydrocarbure de l'invention sont avantageusement des cires comprenant de 18 à 60 atomes de carbone.

La teneur en cire(s) (b) peut varier de 0,5 à 95% en poids, de préférence de 3 à 90% en poids, plus préférentiellement de 3 à 60% en poids, encore plus préférentiellement de 4 à 45% en poids, et encore plus préférentiellement de 5 à 30% en poids, par rapport au poids total de la matrice lipidique constituant les sphéroïdes anhydres de l'invention.

Les sphéroïdes anhydres de l'invention peuvent également comprendre au moins un pigment ou une nacre, de préférence un pigment organique ou inorganique. Le pigment ou la nacre de l'invention peut avoir été traité en surface, c'est-à-dire avoir subi un ou plusieurs traitements de surface de nature chimique, électronique, mécanochimique et/ou mécanique.

La teneur en pigment ou en nacre varie avantageusement de 0 à 20% en poids, et de préférence de 2 à 10% en poids, par rapport au poids total de la matrice lipidique.

La phase continue de la composition de l'invention, non miscible avec les sphéroïdes anhydres, comprend au moins une huile de silicone volatile et/ou non volatile.

Comme huiles de silicone volatiles, on peut citer les silicones volatiles, comme par exemple les huiles de silicones linéaires ou cycliques volatiles, ayant notamment de 2 à 7 atomes de silicium, ces silicones comportant éventuellement des groupes alkyles ou alkoxys comprenant de 1 à 10 atomes de carbone. Comme huiles de silicone volatiles utilisables dans le cadre de l'invention, on peut citer préférentiellement la cyclopentadiméthylsiloxane, l'octaméthyle cyclotétrasiloxane, la décaméthyle cyclopentasiloxane, la dodécaméthyle cyclohexasiloxane, l'heptaméthyle hexyltrisiloxane, l'heptaméthyloctyle trisiloxane, l'hexaméthyle disiloxane, l'octaméthyle trisiloxane, la décaméthyl tétrasiloxane, la dodécaméthyle pentasiloxane, et leurs mélanges.

On peut également citer les huiles linéaires alkyltrisiloxanes volatiles choisies parmi le 3-butyl-1,1,1,3,5,5,5-heptaméthyl trisiloxane, le 3-propyl-1,1,1,3,5,5,5-heptaméthyl trisiloxane, et le 3-éthyl-1,1,1,3,5,5,5-heptaméthyl trisiloxane, et leurs mélanges.

L'huile de silicone volatile préférée est la cyclopentadiméthylsiloxane.

Les huiles de silicone non volatiles utilisables dans la composition selon l'invention peuvent être les polydiméthylsiloxanes (PDMS) comportant au moins un groupement alkyle ou alcoxy comprenant avantageusement de 12 à 24 atomes de carbone, pendant et/ou en bout de chaîne siliconée, les silicones phénylées comme les phényl triméthicones, les phényl diméthicones, les phényl triméthylsiloxy diphénylsiloxanes, les diphényl diméthicones, les diphényl méthyldiphényl trisiloxanes, les 2-phényléthyl triméthylsiloxysilicates.

La phase continue peut en outre comprendre une huile fluorée choisie parmi les huiles fluorosiliconées, les polyéthers fluorés, et les silicones fluorées. On peut notamment utiliser des huiles volatiles fluorées telles que le nonafluorométhoxybutane ou le perfluorométhylcyclopentane.

Selon un mode de réalisation préféré, la phase continue est gélifiée par au moins un agent gélifiant de l'huile de silicone. L'agent gélifiant de l'huile de silicone peut être choisi parmi les silices pyrogénées, les argiles, les hectorites éventuellement modifiées, les esters de dextrine, les esters de glycérol, et les polyamides. Avantageusement, il s'agit d'une silice pyrogénée ou de particules d'aérogel de silice. La silice pyrogénée est modifiée chimiquement en surface, par réaction chimique pour générer une diminution du nombre de groupes silanol présents à la surface de la silice. Les groupes silanol peuvent notamment être remplacés par des groupements hydrophobes tels que :
- des groupements triméthylsiloxyle, qui sont notamment obtenus par traitement de silice pyrogénée en présence d'hexaméthyldisilazane. Des silices ainsi traitées sont dénommées « Silica silylate » selon le CTFA (8ème édition, 2000). Elles sont par exemple commercialisées sous la référence Aerosil R812^{®} par la société EVONIK INDUSTRIES,
- des groupements diméthylsilyloxyle ou polydiméthylsiloxane, qui sont notamment obtenus par traitement de silice pyrogénée en présence de polydiméthylsiloxane ou de diméthyldichlorosilane. Des silices ainsi traitées sont dénommées « Silica dimethyl silylate » selon le CTFA (8ème édition, 2000). Elles sont par exemple commercialisées sous les références Aerosil R972^{®} et Aerosil R974^{®} par la société EVONIK INDUSTRIES.

L'utilisation de l'agent gélifiant de l'huile de silicone permet d'obtenir une phase continue gélifiée. La phase continue gélifiée est de préférence translucide ou transparente. Le terme « translucide » signifie que la phase continue laisse passer les rayons lumineux de manière diffuse, sans permettre de distinguer nettement les contours des sphéroïdes qu'elle contient. Le terme « transparent » signifie quant à lui que la phase continue laisse passer la lumière par réfraction, ce qui permet de distinguer les sphéroïdes qu'elle contient avec netteté. Cette propriété de transparence est particulièrement recherchée quand on disperse des sphéroïdes colorés dans la phase continue siliconée, ce qui permet d'obtenir un effet visuel très attractif.

La phase continue peut également comprendre avantageusement un élastomère de silicone, de préférence préalablement dispersé dans l'huile de silicone. L'élastomère de silicone se présente généralement sous la forme d'un gel, d'une pâte, ou d'une poudre, avantageusement sous la forme d'un gel dans lequel l'élastomère de silicone est dispersé dans une huile hydrocarbonée et/ou une huile siliconée. L'élastomère de silicone est avantageusement choisi parmi le polydiméthylsiloxane (PDMS) (ou diméthicone), le méthyl-polysiloxane (MQ), le vinyl-méthyl-polysiloxane (VMQ), le phényl-vinyl-méthyl-polysiloxane (PVMQ), le fluoro-vinyl-méthyl-polysiloxane (FVMQ), et leurs mélanges. Il peut s'agir plus spécifiquement des gels KSG-15 et KSG-16, commercialisés par Shin-Etsu Silicone, DC 9040 et DC 9041, commercialisés par Dow Corning, et Gransil PC-12, commercialisés par Grant Industries, Inc.

La phase continue peut encre comprendre un polymère filmogène. On entend par polymère filmogène, un polymère apte à former un film continu sur un support. Le polymère filmogène peut être d'origine naturelle ou synthétique, et est avantageusement choisi parmi :
- les triméthylsiloxysilicates,
- les phénylalkylsiloxysilicates dans lesquels le groupe alkyle comprend de préférence de 1 à 6 atomes de carbone, tels que la phénylpropyldiméthylsiloxysilicate,
- les polymères acrylates siliconés tels que les copolymères acrylate/diméthicone, et notamment les copolymères acrylate/diméthicone dans du cyclopentasiloxane (comme par exemple le KP-545 de Shin-Etsu), les copolymères acrylate/diméthicone dans du méthyl triméthicone (comme par exemple le KP-579 de Shin-Etsu), les copolymères acrylate/diméthicone dans de l'isododécane (comme par exemple le KP-550 de Shin-Etsu) ; les copolymères acrylate/polytriméthylsiloxy-méthacrylate, et notamment les copolymères acrylate/polytriméthylsiloxy-méthacrylate dans du diméthicone (comme par exemple le FA-4003 DM de Dow Corning^{®}), les copolymères acrylate/polytriméthylsiloxy-méthacrylate dans de l'isododécane (comme par exemple le FA-4004 ID de Dow Corning^{®}),
- les polyalkylsilsesquioxanes comprenant de 1 à 6 atomes de carbone, et de préférence le polyméthylsilsesquioxane (comme par exemple la Silform^{®} Flexible Resin de Momentive),
- les trialkylsiloxysilylcarbamoyl pullulans dans lesquels le groupe alkyle comprend de 1 à 6 atomes de carbone, et de préférence le triméthylsiloxysilylcarbamoyl pullulan (comme par exemple le TSPL-30-ID de Shin-Etsu),
- les copolymères de la vinylpyrrolidone (VP), et de préférence les copolymères de VP et d'alcène comprenant de 2 à 20 atomes de carbone, tels que les copolymères de VP/eicosène, VP/acétate de vinyle, VP/méthacrylate d'éthyle, VP/méthacrylate d'éthyle/acide méthacrylique, VP/hexadécène, VP/triacontane, VP/styrène, VP/acide acrylique/méthacrylate de lauryle, la polyvinylpyrrolidone (PVP) butylée,
- les copolymères d'un ester vinylique, et de préférence les copolymères acétate de vinyle/stéarate d'allyle, acétate de vinyle/laurate de vinyle, acétate de vinyle/stéarate de vinyle, acétate de vinyle/octadécène, acétate de vinyle/octadécylvinyléther, propionate de vinyle/laurate d'allyle, propionate de vinyle/laurate de vinyle, stéarate de vinyle/octadécène-1, acétate de vinyle/dodécène-1, stéarate de vinyle/éthylvinyléther, propionate de vinyle/cétyl vinyl éther, stéarate de vinyle/acétate d'allyle, diméthyl-2,2-octanoate de vinyle/laurate de vinyle, diméthyl-2,2-pentanoate d'allyle/laurate de vinyle, diméthyl propionate de vinyle/stéarate de vinyle, diméthyl propionate d'allyle/stéarate de vinyle,
- les polyoléfines, hydrogénées ou non hydrogénées, et de préférence les polymères ou copolymères d'alcènes comprenant de 2 à 20 atomes de carbone, tels que les polybutènes, les polyisobutènes, les polydécènes,
- les alkylcelluloses, et de préférence les alkylcelluloses porteurs d'un groupe alkyle comprenant de 2 à 6 atomes de carbone, tels que l'éthylcellulose et la propylcellulose,
- les alcools polyvinyliques, et
- leurs mélanges.

Le polymère filmogène est de préférence choisi parmi les polymères siliconés tels que les triméthylsiloxysilicates ; les phénylalkylsiloxysilicates dans lesquels le groupe alkyle comprend de préférence de 1 à 6 atomes de carbone tels que la phénylpropyldiméthylsiloxysilicate ; et les copolymères de la vinylpyrrolidone.

Selon l'application finale visée, la composition cosmétique anhydre de l'invention peut également contenir des adjuvants habituels dans le domaine cosmétique, tels que des charges ou des conservateurs.

Les charges peuvent être minérales ou organiques, et de toutes formes, plaquettaires, sphériques ou oblongues.

Les charges sont choisies notamment parmi des charges inorganiques telles que le talc, les micas d'origine naturelle ou synthétique, le kaolin, les savons métalliques dérivés d'acides organiques carboxyliques comprenant de 8 à 22 atomes de carbone, et de préférence comprenant de 12 à 18 atomes de carbone, par exemple le stéarate de zinc, le stéarate de magnésium ou le stéarate de lithium, le laurate de zinc, le myristate de magnésium, les oxydes de zinc, les oxydes de titane, le carbonate de calcium, le carbonate de magnésium, l'hydrocarbonate de magnésium, les billes de verre, les billes de céramique, et leurs mélanges.

On peut aussi utiliser comme charges des charges organiques telles que des amidons réticulés ou non, des poudres de polymères de synthèse, réticulées ou non, sphéronisées ou non, expansées ou non, comme des poudres de polyéthylène, des poudres de polyester (par exemple isophtalate ou téréphtalate), des poudres de polyamide (par exemple des poudres de poly-β-alanine et des poudres de nylon comme celles commercialisées sous la dénomination ORGASOL^{®}), des poudres d'acide poly(méth)acrylique ou de poly(méth)acylate telles que les poudres de méthacrylate de méthyle réticulé, des poudres de polyuréthane telles que les copolymères de diisocyanate d'hexaméthylène et de triméthylol hexyl lactone vendus sous les dénominations PLASTIC POWDER^{®} D-400 et PLASTIC POWDER^{®} D-800 par la société TOSHIKI, des poudres de polystyrène réticulé par le divinylbenzène, des poudres de résine de silicone telles que des silsesquioxanes, ou des poudres de tétrafluoroéthylène (Téflon^{®}), et leurs mélanges.

Dans la composition cosmétique de l'invention, le taux en poids des sphéroïdes anhydres peut représenter de 5 à 70% en poids, de préférence de 10 à 60% en poids, et encore plus préférentiellement de 20 à 50% en poids, par rapport au poids total de la composition cosmétique.

Le deuxième objet de l'invention vise un procédé de préparation d'une composition cosmétique selon l'invention comprenant les étapes suivantes :
(i) une étape de préparation d'une matrice lipidique constituant les sphéroïdes anhydres de l'invention par homogénéisation d'au moins une huile hydrocarbonée (a) et éventuellement d'un agent gélifiant de ladite huile hydrocarbonée, et/ou d'au moins une cire (b), sous agitation, à une température allant de 50 à 120°C, afin d'obtenir un mélange liquide et totalement fondu,
(ii) une étape de mise en forme du mélange liquide obtenus à l'étape (i) par dispersion, sous agitation, dans une phase aqueuse chauffée à une température allant de 50 à 120°C,
(iii) une étape de refroidissement brutal de la dispersion obtenue à l'étape (ii), par ajout d'une phase aqueuse ayant de préférence une température inférieure ou égale à 0°C, pour solidifier les gouttelettes obtenues à l'étape (ii) et obtenir des sphéroïdes,
(iv) une étape de séparation par filtration des sphéroïdes obtenus à l'étape (iii), à température ambiante, par exemple à l'aide d'un tamis moléculaire,
(v) une étape de séchage des sphéroïdes obtenus à l'étape (iv), de préférence à température ambiante, pour éliminer l'eau résiduelle,
(vi) une étape de mise en dispersion des sphéroïdes obtenus à l'étape (v), sous agitation, dans une phase continue comprenant au moins une huile de silicone.

La phase continue comprenant au moins une huile de silicone peut être gélifiée par ajout d'un agent gélifiant de l'huile de silicone, avant ou après l'étape de mise en dispersion des sphéroïdes dans la phase continue. La phase continue est alors chauffée à une température allant de 50 à 120°C, de préférence à une température de 85°C, et l'agent gélifiant incorporé dans l'huile de silicone, sous agitation. Une fois la phase continue homogénéisée (absence de grains), celle-ci est refroidie à température ambiante.

Le troisième objet de l'invention vise l'utilisation d'une composition cosmétique selon l'invention pour le maquillage et/ou le soin de la peau du corps ou du visage, et notamment des lèvres.

L'invention vise également un rouge à lèvre et un fond de teint comprenant une composition cosmétique selon l'invention.

Enfin, un dernier objet de l'invention concerne un procédé de maquillage et/ou de soin de la peau du corps ou du visage, et notamment des lèvres, comprenant les étapes suivantes :
(i') prélèvement d'une quantité de composition cosmétique selon l'invention, en quantité nécessaire pour réaliser au moins une application,
(ii') mélange par cisaillement des sphéroïdes anhydres avec la phase continue non miscible comprenant au moins une huile de silicone, contenus dans la composition cosmétique de l'invention, et
(iii') application de la composition cosmétique ainsi mélangé sur la peau du corps ou du visage, et notamment des lèvres.

L'étape de mélange par cisaillement peut être réalisée soit directement sur la peau du corps ou du visage (application directe), par exemple à l'aide des doigts, soit préalablement, au sein d'un dispositif de distribution tel qu'un emballage contenant la composition cosmétique de l'invention (application indirecte). Dans ce dernier cas, la texture de la composition cosmétique peut être distribuée sous forme de sphéroïdes qui sont ensuite écrasés lors de l'application ou modifié de sphéroïdes en crème ou en gel au sein dudit dispositif de distribution, avant application sur la peau.

Outre les dispositions qui précèdent, l'invention comprend encore d'autres dispositions qui ressortiront du complément de description qui suit, qui se rapporte à la préparation de compositions cosmétiques selon l'invention.

### EXEMPLES :

Les différents tests et méthodes de mesure mis en oeuvre dans les exemples sont les suivants :
Mesure du diamètre moyen des sphéroïdes :
   24 heures après fabrication, une dizaine de sphéroïdes sont prélevés. Leurs diamètres sont mesurés à la loupe binoculaire sous un grossissement ×10, à température ambiante. Le diamètre moyen des sphéroïdes représente la moyenne de ces dix mesures.
Mesure du point de fusion :
   Lors de la préparation de la matrice lipidique, un échantillon de cette matrice est versé dans un réservoir percé. Ce réservoir est maintenu à 20°C, sous pression atmosphérique, pendant 24 heures. Il est ensuite placé dans un four, puis chauffé à une vitesse de chauffe de 1°C par minute. Le point de fusion correspond à la fonte de la première goutte de matrice lipidique dans le réservoir.
Test de stabilité :
   La stabilité est évaluée à différentes températures : à 4°C, à température ambiante, et à l'étuve à 50°C, pendant une durée d'un mois.

### Exemple 1 :

**Préparation d'une composition de rouge à lèvres comprenant des sphéroïdes anhydres dans une phase continue siliconée** :

**Tableau 1 : Composition des sphéroïdes (Composition 1)**

| Nom chimique | Nom commercial (fournisseur) | % en poids |
|---|---|---|
| Polyglycéryl-2-triisostéarate | Salacos 43V (Nisshin Oillio Group) | 17,5 |
| Polyisobutène hydrogéné | Parleam^{®} (NOF Corporation) | 12,4 |
| Poly-1-décène hydrogéné | Dekanex 2006 FG (IMCD France) | 11,3 |
| Distéardimonium hectorite | Bentone Gel^{®} PTIS V (Elementis Specialties) | 8,8 |
| Cire synthétique | Lipwax A-4 (Ina Trading) | 7,4 |
| Triméthylolpropane triisostéarate | Salacos 6318V (Nisshin Oillio Group) | 6,6 |
| Méthyl rosinate hydrogénée | Floralyn (PFW Aroma Chemicals) | 6,0 |
| Polybutène | Polybutene M100 (Kemat) | 6,0 |
| Ozokérite | Permulgin 3233 (Koster Keunen) | 4,0 |
| Ester de jojoba | Floraester^{®} 30 (Floratech) | 2,7 |
| Cire de polyéthylène | Jeenate 3H (Jeen International Corporation) | 2,0 |
| Cire d'abeille | Cerabeil blanchie DAB (Baerlocher) | 2,0 |
| Charges | | 2,0 |
| Pigments | | 10,0 |
| Nacre | | 1,2 |
| Antioxydant | | 0,1 |
| **TOTAL** : | | 100,0 |

Tous les ingrédients de la composition 1 sont chauffés à 85°C, sous agitation, jusqu'à obtention d'un mélange homogène. Le mélange ainsi obtenue est ensuite dispersé lentement dans de l'eau à 85°C, tout en maintenant l'agitation, jusqu'à obtention de gouttelettes. La dispersion obtenue est ensuite brutalement refroidie par ajout d'une phase aqueuse à 0°C. L'agitation est stoppée pour éviter d'abîmer les sphéroïdes solidifiés. Les sphéroïdes sont ensuite séparés par filtration, puis séchés à température ambiante.

### Caractérisation des sphéroïdes obtenus :

- Point de fusion : 81°C,
- Diamètre moyen : 2,5 mm.

**Tableau 2 : Composition de la phase continue siliconée (Composition 2)**

| Nom chimique | Nom commercial (fournisseur) | % en poids |
|---|---|---|
| Diphényl diméthicone | KF-54HV (Shin-Etsu) | 80,7 |
| Polydiméthylsiloxane et copolymère acrylate/polytriméthylsiloxyméthacrylate | Dow Corning^{®} FA 4003 DM (Dow Corning) | 12,5 |
| Copolymère VP/eicosène | Unimer U15 (Givaudan Active Beauty) | 5,0 |
| Silice | Aerosil 200 (Evonik Industries) | 1,8 |
| **TOTAL** : | | 100,0 |

La phase continue siliconée de composition 2 est préparée en mélangeant les différents composants listés dans le **Tableau 2.** La diphényl diméthicone (huile de silicone) est chauffée à une température de 85°C, puis la silice (agent gélifiant) est ajoutée doucement dans l'huile de silicone. Le mélange est ensuite maintenu sous forte agitation à une température de 85°C jusqu'à obtention d'un mélange homogène (sans grains). Les deux polymères sont ensuite ajoutés à chaud, à une température de 85°C, et le mélange homogénéisé par agitation. Le mélange est par la suite refroidi à température ambiante jusqu'à obtention d'un gel translucide.

Les sphéroïdes de composition 1 précédemment préparés sont ensuite dispersés dans la phase continue siliconée de composition 2 selon un ratio en poids composition 1/composition 2 de 40/60, sous agitation, à température ambiante.

### Evaluation des propriétés de la composition de rouge à lèvres :

### Test de brillance :

La brillance est mesurée par imagerie polarimétrique. Le test mis en oeuvre consiste à étaler manuellement une quantité précise de la composition (0,15 g) sur une peau synthétique, Bio Skin Plate, sur une surface de 170 × 30 mm. La composition est étalée en bande d'épaisseur homogène, pour obtenir une surface lisse. Trois bandes sont ainsi préparées. Pour chaque mesure, l'échantillon de Bio Skin Plate est déposé sur le support cylindrique d'un appareil SAMBA Hair afin de visualiser l'échantillon. Trois mesures de brillance sont réalisées sur chaque bande, en différents endroits.

Les valeurs de brillance obtenues sont présentées dans le **Tableau 3** ci-après, les résultats étant exprimés en unité de brillance :

**Tableau 3 :**

| Mesures | **Référence gloss** | **Référence rouge à lèvres brillant** | **Référence rouge à lèvres satiné** | **Référence rouge à lèvres mat** | **Composition de l'invention** |
|---|---|---|---|---|---|
| Moyenne | 38,8 | 37,0 | 16,9 | 10,1 | 41,4 |
| Ecart-type | 1,16 | 0,08 | 0,05 | 0,02 | 1,10 |

| | | | | | |
|---|---|---|---|---|---|
| Référence gloss : produit DIOR ADDICT ULTRA-GLOSS (Mintel ID : 4228439) Référence rouge à lèvres brillant : produit DIOR ADDICT LIPSTICK (Mintel ID : 3263269) Référence rouge à lèvres satiné : produit ROUGE DIOR INTERNATIONAL (Mintel ID : 5206147) Référence rouge à lèvres mat : produit DIORIFIC (Mintel ID : 4395479) | | | | | |

La composition de l'invention présente un niveau de brillance supérieur à ceux des rouges à lèvres de référence, et même supérieur à celui d'un gloss.

### Test sensoriel :

Un test sensoriel comparatif entre un rouge à lèvres selon l'invention (sphéroïdes anhydres de composition 1 dans une phase continue siliconée de composition 2) et un rouge à lèvres mat longue tenue de référence (rouge à lèvre MAC MAT LIPSTICK, teinte Russian Red, Mintel ID : 4601379) est ensuite réalisé sur un panel de huit sujets.

L'évaluation des rouges à lèvres s'effectue selon différents critères : glissant, brillance, confort, migration et transfert après 3 heures, tenue de la brillance et du film après 3 heures, selon une échelle de notation allant de 0 (mauvais) à 4 (excellent).

**Tableau 4 :**

| Propriétés évaluées | Rouge à lèvres mat longue tenue de référence | Rouge à lèvres selon l'invention |
|---|---|---|
| Glissant | 2 | 3 |
| Brillance | 1 | 4 |
| Confort | 3 | 3 |
| Migration (après 3h) | 1 | 1 |
| Transfert (après 3h) | 2 | 2 |
| Tenue de la brillance (après 3h) | 0 | 3 |
| Tenue du film (après 3h) | 2 | 2 |

Le rouge à lèvres de l'invention présente un niveau de performances équivalent au rouge à lèvres mat longue tenue de référence en termes de migration, transfert et tenue du film, tout en présentant un pouvoir glissant supérieur, une brillance et une tenue de la brillance très supérieure.

### Exemple 2 :

Quatre compositions de sphéroïdes anhydres A, B, C et D sont préparées.

Les sphéroïdes de compositions A et B correspondent aux sphéroïdes de la demande FR 2 649 608. Ils ont un bas point de fusion de 36-38°C. Un pigment a été ajouté pour favoriser la visualisation de ces sphéroïdes.

Les compositions A, B, C et D sont les suivantes :

**Tableau 5 : Composition A**

| Nom chimique | Nom commercial (fournisseur) | % en poids |
|---|---|---|
| Triglycérides hémi-synthétiques | Pastilles de Lipocire A (Gattefosse) | 99,0 |
| Huile de ricin | | 0,8 |
| Pigment | | 0,2 |
| **TOTAL** : | | 100,0 |

**Tableau 6 : Composition B**

| Nom chimique | Nom commercial (fournisseur) | % en poids |
|---|---|---|
| Ester d'huile d'olive | Phytowax olive 10L 40 (Sophim) | 99,0 |
| Huile de ricin | | 0,8 |
| Pigment | | 0,2 |
| **TOTAL :** | | 100,0 |

**Tableau 7 : Composition C**

| Nom chimique | Nom commercial (fournisseur) | % en poids |
|---|---|---|
| Cire de polyéthylène | Performalène 500 (Baker Hughes) | 7,2 |
| Cire d'abeille | Cerabeil blanchie dab (Univar) | 1,4 |
| Microcrystalline | Microcrystalline wax sp 16 wp (Rossow) | 3,6 |
| Dimère dilinoleyl-dilinoléate | Lusplan DD-DA7 (Unipex) | 7,0 |
| Méthyl rosinate hydrogénée | Floralyn new process (Safic Alcan) | 20,0 |
| Huile d'olive hydrogénée non saponifié | Exolive wax SR (Caroi'Line) | 5,0 |
| Polyisobutène | Parleam^{®} (Rossow) | 10,0 |
| Octyldodécanol | Isofol 20 (IMCD France) | 8,0 |
| Polyglycéryl-2-triisostéarate | | 24,05 |
| Antioxydant | | 0,1 |
| Charges | | 0,5 |
| Pigment | | 12,9 |
| Parfums | | 0,25 |
| **TOTAL** : | | 100,0 |

La composition C comprend 69,05% d'huiles non volatiles (a) (dont 10% d'huile apolaire) et 17,2% de cires (b).

**Tableau 8 : Composition D**

| Nom chimique | Nom commercial (fournisseur) | % en poids |
|---|---|---|
| Cire d'abeille | Candelilla wax sp 75G (Rossow) | 10,0 |
| Cire de riz | Rice wax n°1 (Saci/CFPA) | 1,5 |
| 12-Hydroxyoctadec-9-énoate d'hexadécyle | Ricinoléeate de cétyle (Laboratoires PROD HYG) | 2,0 |
| Triglycéride caprylique/caprique/succinique | Miglyol 829 MB (IMCD France) | 4,0 |
| 2,6,10,15,19,23-Hexaméthyltétracosane | Squalane végétal (Perhydrosqualène) | 10,0 |
| Malate de diisostéaryle | Salacos 222 (Saci/CFPA) | 8,0 |
| Esters de C₁₀₋₃₀ cholestérol/lanostéol | Super stérol ester (Croda) | 3,0 |
| Mélange de propylène carbonate, de stéaralkonium hectorite et d'huile de ricin | Bentone Gel CAO V (Saci/CFPA) | 5,0 |
| Cire d'abeille | Cerabeil blanchie dab (Univar) | 2,6 |
| Ozokérite | Cerozo G 168 (Univar) | 0,5 |
| Huile de ricin | | 29,0 |
| Méthoxycinnamate d'octyle | | 3,0 |
| Antioxydant | | 1,0 |
| Charges | | 2,0 |
| Pigment | | 17,5 |
| Parfums | | 0,9 |
| **TOTAL :** | | 100,0 |

La composition D comprend 57% d'huiles (a) et 19,6% de cires (b).

Les sphéroïdes de composition A, B, C et D ont été préparés selon le même procédé :
- Les ingrédients sont chauffés, mélangés et homogénéisés à 50°C pour les compositions A et B, et à 80°C pour les compositions C et D,
- Lorsque le mélange est homogène, il est dispersé lentement dans une phase aqueuse chauffée à la même température. L'agitation est maintenue jusqu'à obtention de gouttelettes.
- Le mélange est ensuite refroidi brutalement par ajout d'une phase aqueuse refroidie à 0°C. L'agitation est stoppée pour éviter d'abîmer les sphéroïdes solidifiés.
- Les sphéroïdes sont ensuite filtrés, puis séchés à température ambiante.
- Les sphéroïdes sont ensuite incorporés dans une phase continue siliconée de composition H, selon un ratio en poids sphéroïdes anhydres/composition H de 40/60, sous agitation, à température ambiante.

**Tableau 9 : Composition de la phase continue siliconée (Composition H)**

| Nom chimique | Nom commercial (fournisseur) | % en poids |
|---|---|---|
| Diphényl diméthicone | KF-54HV (Shin-Etsu) | 97,0 |
| Silice | Aérosil 200 (Evonik Industries) | 3,0 |
| **TOTAL** : | | 100,0 |

La phase continue siliconée est préparée en chauffant la diphényl diméthicone (huile de silicone) à une température de 85°C, puis en ajoutant doucement la silice (agent gélifiant) dans l'huile de silicone. Le mélange est ensuite maintenu sous forte agitation à une température de 85°C, jusqu'à obtention d'un mélange homogène (sans grains).

### Caractérisation des sphéroïdes obtenus :

Les sphéroïdes anhydres de composition A, B, C et D obtenus sont ensuite caractérisés selon les tests décrits ci-dessus. Les résultats sont rassemblés dans le **Tableau 10** ci-après.

**Tableau 10 :**

| | Point de fusion | Diamètre moyen des sphéroïdes (mm) | Aspect des sphéroïdes | Stabilité |
|---|---|---|---|---|
| Composition A | 37,7°C | 1,7 | Sphéroïdes sphériques | Mauvaise |
| Composition B | 42,5°C | 1,9 | Batonnets | Mauvaise |
| Composition C | 71,9°C | 2,1 | Sphéroïdes sphériques | Bonne |
| Composition D | 68,1°C | 2,0 | Sphéroïdes sphériques | Bonne |

Les sphéroïdes de composition A et B ont un point de fusion inférieur à 50°C, et ne sont pas stables dans le temps. D'autre part, la manipulation des sphéroïdes lors de leur incorporation dans la phase continue siliconée est très délicate : les sphéroïdes ont perdu leur forme et s'agglomèrent en amas, ce qui rend très difficile leur incorporation dans la phase continue siliconée. En outre, les sphéroïdes obtenus ne sont pas réguliers et présentent des formes plus allongées, non reproductibles.

Les sphéroïdes de composition C et D, qui ont un point de fusion supérieur à 50°C, conservent leur forme et leur taille après un mois à l'étuve. Leur manipulation et leur incorporation dans la phase continue siliconée sont aisées. En outre, les sphéroïdes de compositions C et D présentent une bonne stabilité dans le temps. Contrairement aux sphéroïdes de composition A et B, ils s'incorporent facilement dans la phase continue siliconée à l'aide d'une agitation modérée et ne s'agglomèrent pas entre eux.

### Exemple 3 :

**Préparation d'une composition de rouge à lèvres selon l'invention comprenant des sphéroïdes anhydres dans une phase continue siliconée** :

**Tableau 11 : Composition des sphéroïdes (Composition E)**

| Nom chimique | Nom commercial (fournisseur) | % en poids |
|---|---|---|
| Cire d'abeille | Super refined Beeswax PA (Croda) | 8,0 |
| Stéaryl heptanoate - Stéaryl caprylate | PCL Solid (Symrise) | 15,0 |
| Cire d'abeille | Cerabeil Blanchie dab (Baerlocher) | 1,4 |
| Cire de Riz | Rice wax n°1 (Saci/CFPA) | 3,0 |
| 12-Hydroxyoctadec-9-énoate d'hexadécyle | Ricinoléeate de cétyle (Laboratoires PROD HYG) | 2,0 |
| Triglycéride caprylique/caprique/succinique | Miglyol 829 MB (IMCD France) | 4,0 |
| 2,6,10,15,19,23-Hexaméthyltétracosane | Squalane végétal (Perhydrosqualène) | 6,0 |
| Malate de diisostéaryle | Salacos 222 (Saci/CFPA) | 6,0 |
| Esters de C₁₀₋₃₀ cholestérol/lanostéol | Super stérol ester (Croda) | 2,5 |
| Mélange de propylène carbonate, de stéaralkonium hectorite et d'huile de ricin | Bentone Gel CAO V (Saci/CFPA) | 4,0 |
| Azacyclotridécane-2-one | Orgasol^{®} 2002 D NAT COS (Arkema) | 1,0 |
| Calcium aluminium borosilicate | Ronaflake (MERCK) | 8,0 |
| Nacre | Sunshine Fine white C80-3100 (Maprecos) | 5,0 |
| Copolymère VP/eicosène | Unimer U15 (Givaudan Active Beauty) | 5,0 |
| Pigments | | 25,0 |
| Méthoxycinnamate d'octyle | | 3,0 |
| Antioxydant | 0,2 | |
| Parfums | 0,9 | |
| **TOTAL :** | 100,0 | |

Tous les ingrédients de la composition E sont chauffés à 85°C, sous agitation, jusqu'à obtention d'un mélange homogène. Le mélange ainsi obtenu est ensuite dispersé lentement dans de l'eau à 85°C, tout en maintenant l'agitation, jusqu'à obtention de gouttelettes. La dispersion obtenue est ensuite brutalement refroidie par ajout d'une phase aqueuse refroidie à 0°C. L'agitation est stoppée pour éviter d'abîmer les sphéroïdes solidifiés. Les sphéroïdes sont ensuite séparés par filtration, puis séchés à température ambiante.

### Caractérisation des sphéroïdes obtenus :

- Point de fusion : 68,1°C,
- Diamètre moyen : 2,1 mm.

**Tableau 12 : Composition de la phase continue siliconée (Composition F)**

| Nom chimique | Nom commercial (fournisseur) | % en poids |
|---|---|---|
| Diphényl diméthicone | KF-54HV (Shin-Etsu) | 76,1 |
| Silice | Aérosil 200 (Evonik Industries) | 3,0 |
| Décaméthylcyclopentasiloxane | Pentacyclométhicone volatile | 10,0 |
| Cyclopentasiloxane | KF-7312 J (Shin Etsu Chemical Co) | 7,0 |
| Copolymère hexadécène | Unimer U-151 (Givaudan Actifs) | 3,9 |
| **TOTAL** : | | 100,0 |

La phase continue siliconée de composition F est préparée en mélangeant les différents ingrédients listés dans le **Tableau 12.**

La diphényl diméthicone (huile de silicone) est chauffée à une température de 85°C, puis la silice (agent gélifiant) est ajoutée doucement dans l'huile de silicone. Le mélange est ensuite maintenu sous forte agitation à une température de 85°C jusqu'à obtention d'un mélange homogène (sans grains). Les autres ingrédients sont ensuite ajoutés à chaud, à une température de 85°C, et le mélange homogénéisé par agitation. Le mélange est par la suite refroidi à température ambiante jusqu'à obtention d'un gel translucide.

Les sphéroïdes de composition E précédemment préparés sont ensuite mis en dispersion dans la phase continue siliconée de composition F, selon un ratio en poids composition E/composition F de 40/60, sous agitation, à température ambiante.

### Préparation d'une composition comparative G de rouge à lèvres comprenant un mélange des compositions E et F :

La composition cosmétique de l'invention à base de sphéroïdes de composition E dispersés dans une phase continue siliconée de composition F est comparée à une composition cosmétique comparative G dans laquelle les compositions E et F précédemment décrites sont mélangées dans des proportions composition E/composition F de 40/60.

Pour préparer la composition cosmétique comparative G, tous les ingrédients des compositions E et F sont chauffés à 85°C, sous agitation, jusqu'à obtention d'un mélange homogène. Le mélange est ensuite refroidi, sous agitation, à température ambiante.

### Evaluation des propriétés des deux compositions de rouge à lèvres finales :

### Test de stabilité :

La stabilité est évaluée à l'aide du test décrit précédemment. Au bout d'un mois, la composition cosmétique comparative G présente un aspect granuleux avec une couche huileuse à la surface. Au contraire, l'aspect de la composition cosmétique de l'invention n'est pas modifié : les sphéroïdes restent dispersés dans la phase continue siliconée, et la forme des sphéroïdes n'est pas affectée.

### Test de brillance :

La brillance est mesurée à l'aide d'un gloss-mètre Micro-gloss S de BYK Additives and Instruments. Les deux compositions cosmétiques ont été étalées à l'aide d'un barreau cisaillant sur une carte de contraste, avec une épaisseur du film de 40 µm et une vitesse d'étalement de 4 m/s. Après un temps de séchage de 15 minutes à température ambiante, dix mesures ont été réalisées sur l'ensemble de la carte.

Les valeurs de brillance sont présentées dans le **Tableau 13** ci-après, les résultats étant exprimés en unité de brillance :

**Tableau 13 :**

| | Composition cosmétique de l'invention | Composition cosmétique G |
|---|---|---|
| Moyenne | 65,1 | 56,45 |
| Ecart-type | 1,1 | 0,35 |

La composition cosmétique selon l'invention comprenant les sphéroïdes de composition E dispersés dans une phase continue siliconée de composition F présente un niveau de brillance supérieur à la composition cosmétique comparative G.

### Test sensoriel :

Un test sensoriel comparatif entre la composition cosmétique selon l'invention et la composition cosmétique comparative G a été mené sur un panel de dix sujets. Chaque sujet a appliqué chaque composition avec un nombre de passages identiques et a répondu à un questionnaire dans le but d'évaluer la brillance du produit, ainsi que sa tenue.

L'évaluation des deux compositions a été réalisée selon différents critères : brillance, collant à l'application, brillant et tenue après 3 heures, selon l'échelle de notation indiquée au **Tableau 14** :

**Tableau 14 :**

| Note | Brillance | Collant | Tenue |
|---|---|---|---|
| 0 | Ne brille pas | Ne colle pas | Aucune tenue |
| 1 | Brille modérément | Peu collant | Tenue faible |
| 2 | Brille | Moyennement collant | Tenue moyenne |
| 3 | Bonne brillance | Collant | Bonne tenue |
| 4 | Brillance miroir | Très collant | Très bonne tenue |

Les résultats sont exposés dans le **Tableau 15** ci-après :

**Tableau 15 :**

| | Composition cosmétique de l'invention | Composition cosmétique comparative G |
|---|---|---|
| Brillance | 4 | 2 |
| Collant à l'application | 2 | 2 |
| Brillance (après 3h) | 2 | 1 |
| Tenue (après 3h) | 3 | 2 |

La composition cosmétique de l'invention présente globalement un niveau de performance supérieur à la composition cosmétique comparative G.

## Revendications

1. Composition cosmétique anhydre **caractérisée en ce qu'**elle comprend des sphéroïdes anhydres ayant un diamètre de 0,1 à 10 mm et présentant un point de fusion supérieur à 50°C, lesdits sphéroïdes étant constitués d'une matrice lipidique cosmétiquement acceptable, solide à température ambiante, comprenant :
(a) au moins une huile hydrocarbonée, et éventuellement un agent gélifiant de ladite huile hydrocarbonée, et/ou
(b) au moins une cire,
lesdits sphéroïdes étant en dispersion dans une phase continue non miscible comprenant au moins une huile de silicone.

2. Composition cosmétique selon la revendication 1 **caractérisée en ce que** lesdits sphéroïdes sont exempts de tout enrobage externe.

3. Composition cosmétique selon la revendication 1 ou la revendication 2 **caractérisée en ce que** l'huile hydrocarbonée (a) est une huile hydrocarbonée volatile et/ou une huile hydrocarbonée non volatile.

4. Composition cosmétique selon l'une des revendications 1 à 3 **caractérisée en ce que** la matrice lipidique comprend un agent gélifiant de ladite huile hydrocarbonée choisi parmi les silices, les argiles, les hectorites éventuellement modifiées, les esters de dextrine, les polyamides ou les polyamides siliconés, les amides de l'acide L-glutamique ou de l'acide aspartique, les copolymères blocs hydrocarbonés comprenant au moins une unité styrène.

5. Composition cosmétique selon l'une des revendications 1 à 4 **caractérisée en ce que** la matrice lipidique comprend au moins une cire (b) choisie parmi les cires d'hydrocarbures et/ou les cires de polyéthylène, et de préférence parmi les cires d'hydrocarbures.

6. Composition cosmétique selon l'une des revendications 1 à 5 **caractérisée en ce que** ladite matrice lipidique comprend au moins un pigment ou une nacre, et de préférence un pigment organique ou inorganique.

7. Composition cosmétique selon l'une des revendications 1 à 6 **caractérisée en ce que** lesdits sphéroïdes ont un point de fusion allant de 60 à 120°C, et de préférence de 70 à 100°C.

8. Composition cosmétique selon l'une des revendications 1 à 7 **caractérisée en ce que** l'huile de silicone de la phase continue est une huile de silicone volatile et/ou une huile de silicone non volatile.

9. Composition cosmétique selon l'une des revendications 1 à 8 **caractérisée en ce que** la phase continue est gélifiée par au moins un agent gélifiant de l'huile de silicone, ledit agent gélifiant de l'huile de silicone étant de préférence une silice pyrogénée ou des particules d'aérogel de silice.

10. Composition cosmétique selon l'une des revendications 1 à 9 **caractérisée en ce que** lesdits sphéroïdes représentent de 5 à 70% en poids, de préférence de 10 à 60% en poids, et encore plus préférentiellement de 20 à 50% en poids, par rapport au poids total de la composition cosmétique.

11. Procédé de fabrication d'une composition cosmétique selon l'une des revendications 1 à 10 **caractérisé en ce qu'**il comprend les étapes suivantes :
(i) une étape de préparation d'une matrice lipidique constituant les sphéroïdes anhydres par homogénéisation d'au moins une huile hydrocarbonée (a) et éventuellement d'un agent gélifiant de ladite huile hydrocarbonée, et/ou d'au moins une cire (b), sous agitation, à une température allant de 50 à 120°C,
(ii) une étape de mise en forme du mélange liquide obtenu à l'étape (i) par dispersion, sous agitation, dans une phase aqueuse chauffée à une température allant de 50 à 120°C,
(iii) une étape de refroidissement de la dispersion obtenue à l'étape (ii) par ajout d'une phase aqueuse, pour obtenir des sphéroïdes,
(iv) une étape de séparation par filtration des sphéroïdes obtenus à l'étape (iii), à température ambiante,
(v) une étape de séchage des sphéroïdes obtenus à l'étape (iv),
(vi) une étape de mise en dispersion des sphéroïdes obtenus à l'étape (v), sous agitation, dans une phase continue comprenant au moins une huile de silicone.

12. Utilisation d'une composition cosmétique selon l'une des revendications 1 à 10 pour le maquillage et/ou le soin de la peau du corps ou du visage, et notamment des lèvres.

13. Rouge à lèvres **caractérisé en ce qu'**il comprend une composition cosmétique selon l'une des revendications 1 à 10.

14. Fond de teint **caractérisé en ce qu'**il comprend une composition cosmétique selon l'une des revendications 1 à 10.

15. Procédé de maquillage et/ou de soin de la peau du corps ou du visage, et notamment des lèvres, **caractérisé en ce qu'**il comprend les étapes suivantes :
(i') prélèvement d'une quantité de composition cosmétique selon l'une des revendications 1 à 10, en quantité nécessaire pour réaliser au moins une application,
(ii') mélange par cisaillement des sphéroïdes anhydres avec la phase continue non miscible comprenant au moins une huile de silicone, et
(iii') application de la composition cosmétique ainsi mélangé sur la peau du corps ou du visage, et notamment des lèvres.

16. Procédé selon la revendication 15 **caractérisé en ce que** l'étape de mélange par cisaillement est réalisée directement sur la peau du corps ou du visage, par exemple à l'aide des doigts, ou préalablement à l'application sur la peau du corps ou du visage, au sein d'un dispositif de distribution contenant ladite composition cosmétique.

## Patentansprüche

1. Wasserfreie kosmetische Zusammensetzung, **dadurch gekennzeichnet, dass** sie wasserfreie Sphäroide umfasst, die einen Durchmesser von 0,1 bis 10 mm aufweisen und einen Schmelzpunkt über 50 °C aufweisen, wobei die Sphäroide aus einer kosmetisch annehmbaren Fettmatrix bestehen, die bei Umgebungstemperatur fest ist, und Folgendes umfasst:
(a) mindestens ein Kohlenwasserstofföl und gegebenenfalls ein Geliermittel des Kohlenwasserstofföls, und/oder
(b) mindestens ein Wachs,
wobei die Sphäroide in einer kontinuierlichen, nicht mischbaren Phase in Dispersion sind, die mindestens ein Silikonöl umfasst.

2. Kosmetische Zusammensetzung nach Anspruch 1, **dadurch gekennzeichnet, dass** die Sphäroide keinerlei äußere Umhüllung aufweisen.

3. Kosmetische Zusammensetzung nach Anspruch 1 oder Anspruch 2, **dadurch gekennzeichnet, dass** das Kohlenwasserstofföl (a) ein flüchtiges Kohlenwasserstofföl und/oder ein nicht flüchtiges Kohlenwasserstofföl ist.

4. Kosmetische Zusammensetzung nach einem der Ansprüche 1 bis 3, **dadurch gekennzeichnet, dass** die Fettmatrix ein Geliermittel des Kohlenwasserstofföls umfasst, das ausgewählt ist aus Siliziumdioxiden, Tonen, gegebenenfalls modifizierten Hectoriten, Dextrinestern, Polyamiden oder Silikonpolyamiden, Amiden von L-Glutaminsäure oder Asparaginsäure, Kohlenwasserstoff-Blockcopolymeren, die mindestens eine Styroleinheit umfassen.

5. Kosmetische Zusammensetzung nach einem der Ansprüche 1 bis 4, **dadurch gekennzeichnet, dass** die Fettmatrix mindestens ein Wachs (b) umfasst, das ausgewählt ist aus Kohlenwasserstoffwachsen und/oder Polyethylenwachsen und vorzugsweise aus Kohlenwasserstoffwachsen.

6. Kosmetische Zusammensetzung nach einem der Ansprüche 1 bis 5, **dadurch gekennzeichnet, dass** die Fettmatrix mindestens ein Pigment oder ein Perlmutt und vorzugsweise ein organisches oder anorganisches Pigment umfasst.

7. Kosmetische Zusammensetzung nach einem der Ansprüche 1 bis 6, **dadurch gekennzeichnet, dass** die Sphäroide einen Schmelzpunkt zwischen 60 und 120 °C und vorzugsweise zwischen 70 und 100 °C aufweisen.

8. Kosmetische Zusammensetzung nach einem der Ansprüche 1 bis 7, **dadurch gekennzeichnet, dass** das Silikonöl der kontinuierlichen Phase ein flüchtiges Silikonöl und/oder ein nicht flüchtiges Silikonöl ist.

9. Kosmetische Zusammensetzung nach einem der Ansprüche 1 bis 8, **dadurch gekennzeichnet, dass** die kontinuierliche Phase durch mindestens ein Geliermittel des Silikonöls geliert ist, wobei das Geliermittel des Silikonöls vorzugsweise ein pyrogenes Siliziumdioxid oder Siliziumdioxid-Aerogelpartikel ist.

10. Kosmetische Zusammensetzung nach einem der Ansprüche 1 bis 9, **dadurch gekennzeichnet, dass** die Sphäroide zwischen 5 und 70 Gew.-%, vorzugsweise zwischen 10 und 60 Gew.-%, und noch mehr zu bevorzugen zwischen 20 und 50 Gew.-%, bezogen auf das Gesamtgewicht der kosmetischen Zusammensetzung, ausmachen.

11. Verfahren zur Herstellung einer kosmetischen Zusammensetzung nach einem der Ansprüche 1 bis 10, **dadurch gekennzeichnet, dass** es die folgenden Schritte umfasst:
(i) einen Schritt der Vorbereitung einer Fettmatrix, welche die wasserfreien Sphäroide bildet, durch Homogenisierung mindestens eines Kohlenwasserstofföls (a) und gegebenenfalls eines Geliermittels des Kohlenwasserstofföls und/oder mindestens eines Wachses (b) unter Bewegung bei einer Temperatur von 50 bis 120 °C,
(ii) einen Schritt der Formgebung des im Schritt (i) erhaltenen Fettgemischs durch Dispersion unter Bewegung in einer wässrigen Phase, die auf eine Temperatur von zwischen 50 und 120 °C erhitzt wird,
(iii) einen Schritt des Abkühlens der im Schritt (ii) erhaltenen Dispersion durch Beimengung einer wässrigen Phase, um Sphäroide zu erhalten,
(iv) einen Schritt der Trennung durch Filtern der im Schritt (iii) erhaltenen Sphäroide bei Umgebungstemperatur,
(v) einen Schritt des Trocknens der im Schritt (iv) erhaltenen Sphäroide,
(vi) einen Schritt des Dispergierens der im Schritt (v) erhaltenen Sphäroide unter Bewegung in einer kontinuierlichen Phase, die mindestens ein Silikonöl umfasst.

12. Verwendung einer kosmetischen Zusammensetzung nach einem der Ansprüche 1 bis 10 zum Schminken und/oder zur Pflege der Haut des Körpers oder des Gesichts und insbesondere der Lippen.

13. Lippenstift, **dadurch gekennzeichnet, dass** er eine kosmetische Zusammensetzung nach einem der Ansprüche 1 bis 10 umfasst.

14. Make-up, **dadurch gekennzeichnet, dass** es eine kosmetische Zusammensetzung nach einem der Ansprüche 1 bis 10 umfasst.

15. Verfahren zum Schminken und/oder zur Pflege der Haut des Körpers oder des Gesichts und insbesondere der Lippen, **dadurch gekennzeichnet, dass** es die folgenden Schritte umfasst:
(i') Entnehmen einer Menge an kosmetischer Zusammensetzung nach einem der Ansprüche 1 bis 10 in einer Menge, die erforderlich ist, um mindestens ein Auftragen auszuführen,
(ii') Mischen der wasserfreien Sphäroide mit der nicht mischbaren kontinuierlichen Phase, die mindestens ein Silikonöl umfasst, durch Scheren, und
(iii') Auftragen der so gemischten kosmetischen Zusammensetzung auf der Haut des Körpers oder des Gesichts und insbesondere der Lippen.

16. Verfahren nach Anspruch 15, **dadurch gekennzeichnet, dass** der Schritt des Mischens durch Scheren direkt auf der Haupt des Körpers oder des Gesichts, zum Beispiel mittels der Finger, oder vor dem Auftragen auf der Haut des Körpers oder des Gesichts in einer Abgabevorrichtung ausgeführt wird, welche die kosmetische Zusammensetzung enthält.

## Claims

1. An anhydrous cosmetic composition, **characterized in that** it comprises anhydrous spheroids having a diameter of 0.1 to 10 mm and having a melting point above 50°C, said spheroids consisting of a cosmetically acceptable lipid matrix which is solid at room temperature, comprising:
(a) at least one hydrocarbon oil, and optionally a gelling agent for said hydrocarbon oil, and/or
(b) at least one wax,
said spheroids being dispersed in an immiscible continuous phase comprising at least one silicone oil.

2. The cosmetic composition as claimed in claim 1 **characterized in that** said spheroids are free of any external coating.

3. The cosmetic composition as claimed in claim 1 or claim 2, **characterized in that** the hydrocarbon oil (a) is a volatile hydrocarbon oil and/or a non-volatile hydrocarbon oil.

4. The cosmetic composition as claimed in one of claims 1 to 3, **characterized in that** the lipid matrix comprises a gelling agent for said hydrocarbon oil selected from silicas, clays, optionally modified hectorites, dextrin esters, silicone polyamides or polyamides, amides of L-glutamic acid or of aspartic acid, hydrocarbon block copolymers comprising at least one styrene unit.

5. The cosmetic composition as claimed in one of claims 1 to 4, **characterized in that** the lipid matrix comprises at least one wax (b) selected from hydrocarbon waxes and/or polyethylene waxes, and preferably from hydrocarbon waxes.

6. The cosmetic composition as claimed in one of claims 1 to 5, **characterized in that** said lipid matrix comprises at least one pigment or nacre, and preferably an organic or inorganic pigment.

7. The cosmetic composition as claimed in one of claims 1 to 6, **characterized in that** said spheroids have a melting point ranging from 60 to 120°C, and preferably from 70 to 100°C.

8. The cosmetic composition as claimed in one of claims 1 to 7, **characterized in that** the silicone oil of the continuous phase is a volatile silicone oil and/or a non-volatile silicone oil.

9. The cosmetic composition as claimed in one of claims 1 to 8, **characterized in that** the continuous phase is gelled by at least one silicone oil gelling agent, said silicone oil gelling agent preferably being a pyrogenic silica or silica aerogel particles.

10. The cosmetic composition as claimed in one of claims 1 to 9, **characterized in that** said spheroids represent from 5 to 70% by weight, preferably from 10 to 60% by weight, and even more preferentially from 20 to 50% by weight, relative to the total weight of the cosmetic composition.

11. A process for manufacturing a cosmetic composition as claimed in one of claims 1 to 10, **characterized in that** it comprises the following steps:
(i) a step of preparing a lipid matrix constituting the anhydrous spheroids by homogenizing at least one hydrocarbon oil (a) and optionally a gelling agent for said hydrocarbon oil, and/or at least one wax (b), with stirring, at a temperature ranging from 50 to 120°C,
(ii) a step of shaping the liquid mixture obtained in step (i) by dispersion, with stirring, in an aqueous phase heated to a temperature ranging from 50 to 120°C,
(iii) a step of cooling the dispersion obtained in step (ii) by adding an aqueous phase, to obtain spheroids,
(iv) a step of separating by filtration the spheroids obtained in step (iii), at room temperature,
(v) a step of drying the spheroids obtained in step (iv),
(vi) a step of dispersing the spheroids obtained in step (v), with stirring, in a continuous phase comprising at least one silicone oil.

12. Use of a cosmetic composition as claimed in one of claims 1 to 10 for make-up and/or care of the skin of the body or face, and in particular the lips.

13. A lipstick **characterized in that** it comprises a cosmetic composition as claimed in one of claims 1 to 10.

14. A foundation **characterized in that** it comprises a cosmetic composition as claimed in one of claims 1 to 10.

15. A process for make-up and/or care of the skin of the body or face, and in particular the lips, **characterized in that** it comprises the following steps:
(i') collecting a quantity of the cosmetic composition as claimed in one of claims 1 to 10, in the quantity necessary to carry out at least one application,
(ii') shear mixing the anhydrous spheroids with the immiscible continuous phase comprising at least one silicone oil, and
(iii') applying the cosmetic composition thus mixed to the skin of the body or face, and in particular the lips.

16. The process as claimed in claim 15, **characterized in that** the shear mixing step is carried out directly on the skin of the body or face, for example using the fingers, or prior to application to the skin of the body or face, within a dispensing device containing said cosmetic composition.
